(19) Europäisches Patentamt
European Patent Office
Office européen des brevets

(11) EP 0 998 497 B1

(12) **EUROPÄISCHE PATENTSCHRIFT**

(45) Veröffentlichungstag und Bekanntmachung des Hinweises auf die Patenterteilung:
**13.06.2001 Patentblatt 2001/24**

(21) Anmeldenummer: **98942535.0**

(22) Anmeldetag: **10.07.1998**

(51) Int Cl.$^7$: **C08B 13/00**, C08B 11/20

(86) Internationale Anmeldenummer:
**PCT/EP98/04301**

(87) Internationale Veröffentlichungsnummer:
**WO 99/05178 (04.02.1999 Gazette 1999/05)**

(54) **WASSERLÖSLICHE, FLOCKBARE UND BIOLOGISCH ABBAUBARE HYDROXYALKYLCELLULOSE-2-HYDROXYCARBONSÄUREESTER**

WATER-SOLUBLE BIODEGRADABLE HYDROXYALKYL CELLULOSE-2-HYDROXYCARBOXYLIC ACID ESTERS WHICH CAN FLOCCULATE

ESTERS D'ACIDE HYDROXYALKYLCELLULOSE-2-HYDROYCARBOXYLIQUE HYDROSOLUBLES, FLOCULABLES ET BIODEGRADABLES

(84) Benannte Vertragsstaaten:
**AT BE DE FI FR GB IT NL SE**

(30) Priorität: **23.07.1997 DE 19731575**

(43) Veröffentlichungstag der Anmeldung:
**10.05.2000 Patentblatt 2000/19**

(73) Patentinhaber: **Wolff Walsrode AG**
**29655 Walsrode (DE)**

(72) Erfinder:
- **SIMON, Joachim**
  **D-40589 Düsseldorf (DE)**
- **MÜLLER, Hanns-Peter**
  **D-51519 Odenthal (DE)**
- **KOCH, Rainhard**
  **D-51065 Köln (DE)**
- **MÜLLER, Volkhard**
  **D-29699 Bomlitz (DE)**
- **ENGELHARDT, Jürgen**
  **D-51373 Leverkusen (DE)**
- **SZABLIKOWSKI, Klaus**
  **D-29664 Walsrode (DE)**
- **KOCH, Wolfgang**
  **D-29699 Bomlitz (DE)**

(74) Vertreter: **Pettrich, Klaus-Günter, Dr. et al**
**c/o Bayer AG,**
**Konzernbereich RP**
**Patente und Lizenzen**
**51368 Leverkusen (DE)**

(56) Entgegenhaltungen:
**EP-A- 0 626 392** **EP-A- 0 732 341**
**EP-A- 0 806 433** **US-A- 3 455 714**

- **DATABASE WPI Week 8026 Derwent Publications Ltd., London, GB; AN 80-45743c XP002087681 "Anionic cellulose deriv. prodn.- by reaction of alkali cellulose, alkylene oxide and lactone or sultone" & JP 55 065201 A (FUJI CHEM CO LTD) , 16. Mai 1980**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue wasserlösliche, verdickende, thermoplastische, filmbildende und biologisch schnell abbaubare Celluloseether-Lactate.

Die biologische Abbaubarkeit eines Polysaccharidderivats ist abhängig von der Höhe des Substitutionsgrades jeder Saccharideinheit [siehe j.G. Batelaan in The Handbook of Environmental Chemistry, Volume 3, Part F, Ed. O. Hutzinger, Springer Verlag, 1992, 229-336; M.G. Wirick, Journal of Polymer Science, Part A-1, 6 (1968), 1705-1718]. So sind alle technisch verfügbaren Cellulosederivate nur mit durchschnittlichen Substitutionsgraden kleiner als 1,0 biologisch schnell abbaubar. Nicht ionische Celluloseether werden jedoch erst mit durchschnittlichen Substitutionsgraden >>1 wasser-löslich. Die bekannten Konsistenzregler für wässrige Systeme auf Basis nicht ionischer Celluloseether wie z. B. Methylcellulose und Hydroxyethylcellulose weisen somit Substitutionsgrade >1,5 auf. Verdicker für organische Lösungsmittel wie z.B. Hydroxypropylcellulose sogar Substitutionsgrade >2. Solche Verbindungen sind nur schlecht biologisch abbaubar (harter CSB).

Aufgabe der vorliegenden Erfindung ist die Enwicklung einer großtechnisch umsetzbaren Synthese wasser- und/oder organolöslicher Cellulose-Derivate, die schnell und vollständig biologisch abbauen.

Erfindungsgemäß gelingt dies durch Umsetzung von Hydroxyalkylcelluloseethern mit einem molekularen Substitutionsgrad mit Hydroxyalkylgruppen kleiner als 1,5 ($MS_{Hydroxyalkyl}$<1,5) zu Hydroxyalkylcellulose-2-hydroxycarbonsäureestern mit einem molekularen Substitutionsgrad mit 2-Hydroxycarbonsäure-Gruppen größer als 0,4 und kleiner als 3 (0,4<$MS_{2\text{-}Hydroxycarbonsäure}$<3).

Cellulose-Lactate sind bereits bekannt. In der DE 33 22 118 werden Celluloseester beschrieben, die durch Umsetzung von Cellulose mit Lactid oder Glycolid in cellulosespezifisschen Lösungsmittelsystemen wie Dimethylacetamid/LiCl hergestellt werden. Nach dieser Methode gelingt die Synthese niedrig substituierter, wasserlöslicher Cellulose-2-hydroxycarbonsäureester, die als Beschichtungsmittel oder Konsistenzregler eingesetzt werden können. Allerdings ist der Einsatz des Lösungsmittelsystems Dimethylacetamid/Lithiumchlorid aufwendig und groß-technisch nicht praktikabel.

Es wurde festgestellt, daß Hydroxyalkylcellulosen mit Substitutionsgraden kleiner als 1 in den üblichen organischen Lösungsmitteln wie z. B. Dioxan, Dimethylacetamid N-Methylpyrrolidon oder *tert*.-Butanol mit Glycolid oder Lactid, dem cyclischen Dimeren der Milchsäure zu wasserlöslichen Hydroxyalkylcellulose-2-hydroxycarbonsäureestern umgesetzt werden können. Spezifische Lösungsmittelsysteme für Cellulose wie z. B. N-Methylmorpholinoxid oder Dimethylacetamid/Lithiumchlorid werden hierfür nicht benötigt. Die zugrundeliegenden Hydroxyalkylcelluloseether werden gemäß dem Stand der Technik durch Alkalisierung eines Zellstoffs mit wässriger Natronlauge und anschließende Veretherung mit Alkylenoxiden hergestellt.

Die erfindungsgemäßen Hydroxyalkylcellulose-2-hydroxycarbonsäureester bauen trotz ihres hohen Gesamtsubstitutionsgrades in wäßriger Lösung biologisch deutlich schneller ab als Alkyl- und Hydroxyalkylcelluloseether mit vergleichbaren Gesamtsubstitutionsgraden. Die erfindungsgemäß herzustellenden Hydroxyalkyl-cellulose-2-hydroxycarbonsäureester lassen sich durch die allgemeine Struktur (I)

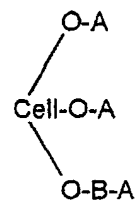

(I)

beschreiben, wobei Cell-O den substituierten Rest einer Hydroxylgruppe an der Cellulose-Kette repräsentiert und die Gruppen A eine monomere oder oligomere 2-Hydroxycarbonsäure der Struktur (II) ist

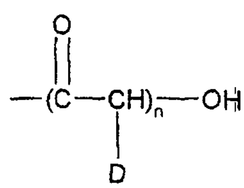

(II)

worin D für Wasserstoff (-H) oder Methyl ($-CH_3$) steht und n eine ganze Zahl zwischen 0 und 10 ist. B ist eine polymere Ethergruppe der allgemeinen Struktur (III)

$$—(E—O)_n— \qquad (III)$$

worin E für eine verzweigte oder unverzweigte Kohlenstoffkette mit 2 bis 6 C-Atomen steht und n eine ganze Zahl zwischen 0 und 10 ist. Für alle Gruppen A, die nicht der Struktur (II) entsprechen, gilt, daß A Wasserstoff (-H) ist.

Zur Synthese dieser Hydroxyalkylcellulose-2-hydroxycarbonsäureester wird der Hydroxyalkylcelluloseether in einem organischem Lösungsmittel suspendiert und mit einem reaktiven 2-Hydroxycarbonsäure-Derivat versetzt. Die Reaktionszeit beträgt 1 bis 10 Stunden bei Reaktionstemperaturen zwischen 50°C und 150°C, bevorzugt zwischen 80°C und 130°C.

Geeignete Celluloseether sind z. B. Hydroxyethylcellulose, Hydroxypropylcellulose, Hydroxybutylcellulose und deren Mischether mit Substitutionsgraden ($MS_{Hydroxyalkyl}$) zwischen 0,1 und 1,5, bevorzugt zwischen 0,5 und 1,0.

Geeignete 2-Hydroxycarbonsäure-Derivate sind die cyclischen Dimeren Glycolid, L-, D- und *meso*-Lactid sowie Milchsäure-Ester und Milchsäure-Oliomere mit 2 bis 10 Wiederholungseinheiten.

Als Suspensions- bzw. Lösungsmittel eignen sich polare aprotische Verbindungen wie z. B. Dimethylformamid, Dimethylacetamid, Dioxan, Tetrahydrofuran, N-Methylmorpholin, N-Methylpyrrolidon, Dimethoxymethan, Dimethylether, Diethylen-glycoldimethylether, des weiteren protische Lösungsmittel wie z. B. tert.-Butanol. Die Umsetzung kann auch lösungsmittelfrei in einer Lactid-Schmelze erfolgen. Die erfindungsgemäßen Celluloseetherester sind wasserlöslich und gute Filmbildner. Bei steigendem Substitutionsgrad mit Hydroxyalkyl- und 2-Hydroxycarbonsäuregruppen werden die Produkte zusätzlich organolöslich und thermoplastisch verarbeitbar.

Hydroxyethyl- und Hydroxypropylcellulose-Lactate, deren Summe der Substitutionsgrade mit Hydroxyalkyl- und Milchsäuregruppen 1,5 übersteigt ($\Sigma$ $MS_{Lactat}$ und $MS_{Hydroxyalkyl}$ >1,5), sind nur unterhalb einer bestimmten Temperatur wasserlöslich. Oberhalb dieser Temperatur (Flocktemperatur) fallen die Hydroxyalkylcellulose-Lactate aus einer wäßrigen Lösung aus. Diese Flocktemperatur ist abhängig von der Substitutionshöhe der Celluloseetherester. Durch Variation der Substitutionsgrade mit Lactat- und Hydroxyalkyl-Gruppen kann ein thermischer Flockpunkt der Hydroxyalkylcellulose-Lactate zwischen 30°C und 75°C eingestellt werden. Bei Substitutionsgraden mit Lactat-Gruppen größer als 3 ($MS_{Lactat}$ >3) sinkt der Flockpunkt unterhalb Raumtemperatur. Die Produkte werden dann sowohl in kaltem als auch in heißem Wasser unlöslich.

Die Produkte können überall dort zur Anwendung kommen, wo wasserlösliche und biologisch abbaubare Produkte einsetzbar sind. So z. B. als Konsistenzregler für Lebensmittel, Kosmetika, Baustoffe, Farben und Abbeizpasten oder zur Herstellung von Mikrokapseln für die Bereiche Lebensmittel, Pflanzenschutz und Pharma.

Die Substitutionsgrade lassen sich mit den üblichen Methoden der Cellulose-derivatanalytik wie z. B. Zeiselspaltung, Elementaranalyse, Gaschromatographie und $^{13}$C-NMR-Spektroskopie bestimmen. Die erfindungsgemäß beanspruchte Eigenschaft der biologischen Abbaubarkeit wird mit dem Zahn-Wellens-Test, DIN EN 29.888 untersucht.

Der Gegenstand der vorliegenden Erfindung soll anhand der vorliegenden Beispiele noch näher erläutert werden.

**Beispiel 1**

Eine Hydroxypropylcellulose (75 g / 0,36 mol) mit einem molaren Substitutionsgrad ($MS_{Hydroxypropyl}$) von 0,92 wird in 691 ml Dimethylacetamid suspendiert und auf 130°C geheizt. Nach Zugabe von 25 g (0,18 mol) L-Lactid rührt man 5 h. Die resultierende pastöse Masse wird in Aceton eingerührt, das ausfallende Produkt isoliert, gewaschen und getrocknet. Man erhält 89 g Hydroxypropylcellulose-Lactat.

| | |
|---|---|
| $MS_{Lactat}$ | 0,6 |
| $V_2$(Wasser) | 413 mPas |
| Biologischer Abbau | 78 % |
| Erweichungspunkt | 170°C |

**Beispiel 2**

Eine Hydroxypropylcellulose (75 g / 0,36 mol) mit einem molaren Substitutionsgrad ($MS_{Hydroxypropyl}$) von 0,92 wird in 691 ml Dimethylacetamid suspendiert und auf 130°C geheizt. Nach Zugabe von 75 g (0,52 mol) L-Lactid rührt man 5 h. Die resultierende pastöse Masse wird in Aceton eingerührt, das ausfallende Produkt isoliert, gewaschen und getrocknet. Man erhält 100 g Hydroxypropylcellulose-Lactat.

| | |
|---|---|
| $MS_{Lactat}$ | 1,0 |
| $V_2$ (Wasser) | 307 mPas |
| Flockpunkt | 59°C |
| Biologischer Abbau | 71% |

**Beispiel 3**

Eine Hydroxypropylcellulose (75 g / 0,36 mol) mit einem molaren Substitutionsgrad ($MS_{Hydroxypropyl}$) von 0,92 wird in 691 ml Dimethylacetamid suspendiert und auf 130°C geheizt. Nach Zugabe von 104 g (0,72 mol) L-Lactid rührt man 5 h. Die resultierende pastöse Masse wird in Aceton eingerührt, das ausfallende Produkt isoliert, gewaschen und getrocknet. Man erhält 110 g Hydroxypropylcellulose-Lactat.

| | |
|---|---|
| $MS_{Lactat}$ | 1,5 |
| $V_2$(Wasser) | 153 mPas |
| Flockpunkt | 35°C |
| Biologischer Abbau | 80% |
| Erweichungspunkt | 170°C |

**Beispiel 4**

Wäßrige Milchsäure (85 %) wird bei Temperaturen > 180°C unter Anlegen eines Vakuums über 4 Stunden zu Oligomeren kondensiert. Danach beträgt die Säurezahl <100 entsprechend Oligomeren der Milchsäure mit mittleren Oligomerisierungs-graden von 4 - 8. 10,8 g der Milchsäure-Oligomeren wurden mit 8 g (0,04 mol) einer Hydroxypropylcellulose mit einem molaren Substitutionsgrad von 0,82 ($MS_{HP}$0,82) in DMAc bei 120°C umgesetzt. Nach Entfernen des Lösungsmittels isoliert man 14 g HPC-Lactat.

| | |
|---|---|
| $MS_{Lactat}$ | 2,0 |
| Erweichungspunkt | 170°C |

**Beispiel 5**

Eine Hydroxyethylcellulose (75 g / 0,40 mol) mit einem molaren Substitutionsgrad ($MS_{Hydroxyethyl}$) von 0,68 wird in 691 ml N-Methylpyrrolidon suspendiert und auf 130°C geheizt. Nach Zugabe von 87 g (0,60 mol) L-Lactid rührt man 5 h. Die resultierende pastöse Masse wird in Aceton eingerührt und das ausfallende Produkt isoliert, gewaschen und getrocknet. Man erhält 90 g Hydroxyethylcellulose-Lactat.

| | |
|---|---|
| $MS_{Lactat}$ | 1,8 |
| $V_2$(Wasser) | 956 mPas |
| Flockpunkt | 35°C |
| Biologischer Abbau | 80% |

**Vergleichsbeispiele**

| Hydroxyethylcellulose | |
|---|---|
| $MS_{Hydroxycthyl}$ | 2,5 |
| Biologischer Abbau | 13% |

| Hydroxypropylcellulose | |
|---|---|
| $MS_{Hydroxypropyl}$ | 4,0 |
| Biologischer Abbau | 22 % |

**Patentansprüche**

1. Wasserlösliche und biologisch abbaubare Hydroxyalkylcellulose-2-hydroxycarbonsäureester mit. einem molekularen Substitutionsgrad der Hydroxyalkylgruppe kleiner als 1,5 ($MS_{Hydroxyalkyl}$<1,5) und einem molekularen Substitutionsgrad der 2-Hydroxycarbonsäure-Gruppe größer als 0,4 und kleiner als 3 (0,4<$MS_{2\text{-}Hydroxycarbonsäure}$<3).

2. Wasserlösliche und biologisch abbaubare Hydroxyalkylcellulose-2-hydroxycarbonsäureester gemäß Anspruch 1, wobei die Hydroxyalkylgruppe Hydroxyethyl, Hydroxypropyl oder Hydroxybutyl- und die 2-Hydroxycarbonsäuregruppe monomeres Oder oligomeres Glycolat, D- oder L-Lactat ist.

3. Wasserlösliche und biologisch abbaubare Hydroxypropylcellulose-Lactate gemäß Anspruch 2, dadurch gekennzeichnet, daß die Summe aus den molekularen Substitutionsgraden mit Lactat- und mit Hydroxypropylgruppen größer als 1,5 ist (Σ $MS_{Lactat}$ und $MS_{Hydroxypropyl}$ >1,5) und dass diese Produkte in heißem Wasser unlöslich sind.

4. Verfahren zur Herstellung der Celluloseetherester gemäß Anspruch 1 durch Umsetzung eines Hydroxyalkylcelluloseethers ($MS_{Hydroxyalkyl}$ <1,5) mit den cyclischen 2-Hydroxycarbonsäuredimeren Glycolid, L-, D- bzw. *meso*-Lactid sowie Milchsäure-Ester und Milchsäure-Oliomere mit 2 bis 10 Wiederholungseinheiten.

5. Verwendung der Hydroxyalkylcellulose-2-hydroxycarbonsäureester gemäß einem der Ansprüche 1 bis 3 als Konsistenzregler für Kosmetika, Baustoffe, Farben und Abbeizpasten, sowie zur Herstellung von Mikrokapseln.

**Claims**

1. Water-soluble, biodegradable hydroxyalkyl cellulose-2-hydroxycarboxylic acid esters with a molecular degree of substitution of the hydroxyalkyl group of less than 1.5 ($MS_{hydroxyalkyl}$ <1.5) and a molecular degree of substitution of the 2-hydroxycarboxylic acid group greater than 0.4 and less than 3 (0.4 < $MS_{2\text{-hydroxycarboxylic acid}}$ <3).

2. Water-soluble, biodegradable hydroxyalkyl cellulose-2-hydroxycarboxylic acid esters according to claim 1, wherein the hydroxyalkyl group is hydroxyethyl, hydroxypropyl or hydroxybutyl and the 2-hydroxycarboxylic acid group is monomeric or oligomeric glycolate, D- or L-lactate.

3. Water-soluble, biodegradable hydroxypropyl cellulose lactates according to claim 2, characterised in that the sum of the molecular degrees of substitution with lactate and hydroxypropyl groups is greater than 1.5 (Σ $MS_{lactate}$ and $MS_{hydroxypropyl}$ >1.5) and in that these products are insoluble in hot water.

4. Process for the production of the cellulose ether esters according to claim 1 by reacting a hydroxyalkyl cellulose ether ($MS_{hydroxyalkyl}$ <1.5) with the cyclic 2-hydroxycarboxylic acid dimers glycolide, L-, D- or *meso*-lactide as well as lactic acid esters and lactic acid oligomers with 2 to 10 repeat units.

5. Use of the hydroxyalkyl cellulose-2-hydroxycarboxylic acid esters according to one of claims 1 to 3 as consistency regulators for cosmetics, building materials, paints and strippers as well as for producing microcapsules.

**Revendications**

1. Esters d'acides 2-hydroxycarboxyliques d'hydroxyalkylcellulose solubles dans l'eau et biodégradables, ayant un degré de substitution moléculaire par le groupe hydroxyalkyle inférieur à 1,5 ($MS_{hydroxyalkyle} < 1,5$) et un degré de substitution moléculaire par le groupe acide 2-hydroxycarboxylique supérieur à 0,4 et inférieur à 3 ($0,4 < MS_{acide\ 2\text{-}hydroxycarboxylique} < 3$).

2. Esters d'acides 2-hydroxycarboxyliques d'hydroxyalkylcellulose solubles dans l'eau et biodégradables selon la revendication 1, dans lesquels le groupe hydroxyalkyle est un groupe hydroxyéthyle, hydroxypropyle ou hydroxybutyle et le groupe acide 2-hydroxycarboxylique est un glycolate ou un D-ou L-lactate monomère ou oligomère.

3. Lactates d'hydroxypropylcellulose solubles dans l'eau et biodégradables selon la revendication 2, caractérisés en ce que la somme des degrés de substitution moléculaire par les groupes lactate et hydroxypropyle est supérieur à 1,5 ($\Sigma\ MS_{lactate}$ et $MS_{hydroxypropyle} > 1,5$) et en ce que ces produits sont insolubles dans l'eau chaude.

4. Procédé de préparation des esters d'éthers de cellulose selon la revendication 1 par réaction d'un éther d'hydroxyalkylcellulose ($MS_{hydroxyalkyle}<1,5$) avec les dimères cycliques d'acides 2-hydroxycarboxyliques qui sont le glycolide, le L-, le D- ou le mésolactide, ainsi qu'avec des esters de l'acide lactique et des oligomères de l'acide lactique contenant 2 à 10 unités répétitives.

5. Utilisation des esters d'acides 2-hydroxycarboxyliques d'hydroxyalkylcellulose selon l'une des revendications 1 à 3 comme agents de réglage de la consistance pour des cosmétiques, des matériaux de construction, des peintures et des pâtes décapantes, ainsi que pour la préparation de microcapsules.